# EUROPEAN PATENT SPECIFICATION

(11) **EP 4 306 066 B1**
(45) Date of publication and mention of the grant of the patent: **09.04.2025**
(21) Application number: 23201042.1
(22) Date of filing: 08.04.2020
(51) Int. Cl.: A61B 17/70

(54) **INTERSPINOUS VERTEBRAL DISTRACTOR**
INTERSPINALER WIRBELDISTRAKTOR
DISTRACTEUR VERTÉBRAL INTERÉPINEUX

(43) Date of publication of application: 17.01.2024
(62) Divisional of application: 20727366.5
(73) Proprietor: Diametros Medical S.r.l., 00135 Roma (IT)
(72) Inventor: GARLATTI, Giovanni, San Casciano in Val di Pesa (IT); FORTUNA, Lorenzo, PELAGO (IT)
(74) Representative: Lunati & Mazzoni S.r.L.

(56) References cited:
- WO-A1-2012/069877
- US-A1- 2007 225 807
- US-A1- 2009 254 185
- US-A1- 2016 206 352

## Description

This invention concerns an interspinous distraction device of the type specified in the preamble of the first claim.

In particular, the invention relates to an implant designed to be implanted, preferably percutaneously, in an interspinous space to space two adjacent vertebrae apart.

As is well known, interspinous distraction can be surgically achieved by introducing an interspinous vertebral distractor between two spinous processes that is controlled by an operator-controlled device for inserting and controlling the distractor.

Distractors are prostheses designed to be implanted in the space between the spinous processes of two adjacent vertebrae, in order to maintain an intervertebral distraction that is suitable for limiting the loads transmitted between the vertebrae in case of intervertebral disc degenerative diseases, thus limiting associated pain.

They mainly consist of a base body that can be arranged between the spinous processes and the distractor's expansion anchoring means. The expansion of the anchoring means is controlled by the control device.

The interspinous vertebral distraction procedure consists in positioning a distractor in the space between the spinous processes of two adjacent vertebrae. In detail, with the patient in the prone position, the affected intervertebral space is identified, the spinous processes are brought closer through expansion cannulae, the measurement of the distractor to be inserted is determined by means of a probe and, finally, the selected distractor is inserted.

A first example of a procedure and distraction device and, in particular, a distractor is described in WO2006102269. In this case the distractor has a first ogive end so as to penetrate the intervertebral space, which is formed by a first pair of wings that open; the opposite end is equipped with a second pair of fixed wings enclosing the column between the two pairs of wings.

Another example is described in document US8998955 wherein there are two pairs of mobile wings. Each wing has one end hinged to the base body and the other end is free. To carry out the locking, this patent prescribes rotating the wings at angles greater than 120° so as to have the free ends facing the vertebrae.

An additional example is described in WO2012069877 where locking and the relative ease of introduction and extraction are achieved by means of hooked wings. One more example is described in US2007225807.

The described prior art comprises some significant drawbacks.

In particular, despite the solutions outlined above, the interspinous vertebral distraction procedure/device, and, in particular, the distractor, are relatively complicated and, therefore, particularly expensive. This is mainly due to the fact that during the procedure, the operator encounters particular difficulties in inserting and defining a stable positioning of the interspinous vertebral distractors.

Another drawback is the low stability of the interspinous vertebral distractors once they have been implanted.

An equally important drawback is the fact that the control kinematics of the mobile wings is particularly complex.

Another drawback is, thus, the costs and difficulties in implementing the known interspinous vertebral distractors.

In this context, the technical task underlying this invention is to devise an interspinous distraction device able to substantially overcome at least some of the drawbacks mentioned above.

Said technical task comprises the important purpose of the invention's having an interspinous vertebral distractor that, once implanted, is easy to insert and stable, and that is easy to implement and, therefore, costs less.

The technical task and the specified purposes are achieved by an interspinous distraction device as claimed in Claim 1. Preferred embodiments are described in the dependent claims.

The characteristics and advantages of the invention are clarified by the following detailed description of preferred embodiments thereof, with reference to the accompanying drawings, wherein:
**Fig. 1** shows, to scale, an interspinous vertebral distractor according to the invention;
**Figs. 2a-2e** illustrate, to scale, the distractor in a sequence during an interspinous vertebral distraction procedure;
**Fig. 3** shows, to scale, a cross-section of the distractor in Fig. 1;
**Fig. 4** shows, to scale, an assembly of an interspinous vertebral distraction device according to the invention;
**Fig. 5** illustrates, to scale, the assembly in Fig. 4;
**Figs. 6a-6f** represent, to scale, a preparation sequence for the interspinous vertebral distraction device according to the invention; and
**Fig. 7** schematises the interspinous vertebral distraction.

In this document, the measures, values, shapes, and geometric references (such as perpendicularity and parallelism), when used with words like "about" or other similar terms such as "approximately" or "substantially", are to be understood as except for measurement errors or inaccuracies due to production and/or manufacturing errors and, above all, except for a slight divergence from the value, measure, shape, or geometric reference which it is associated with. For example, if associated with a value, such terms preferably indicate a divergence of no more than 10% of the value itself.

Furthermore, when used, terms, such as "first", "second", "higher", "lower", "main", and "secondary" do not necessarily identify an order, relationship priority, or relative position, but they can simply be used to distinguish different components more clearly from one another.

The measurements and data provided in this text are to be considered as performed in ICAO International Standard Atmosphere (ISO 2533), unless otherwise indicated.

With reference to the figures, the reference number 1 indicates, as a whole, the interspinous vertebral distractor according to the invention.

The distractor 1 is a percutaneous device that can be implanted for interspinous distraction. It is designed to be implanted in an interspinous space so that it is interposed between two adjacent vertebrae and, thus, spaces two spinous processes apart.

The distractor 1 defines a longitudinal axis **1a** that is, suitably, barycentric.

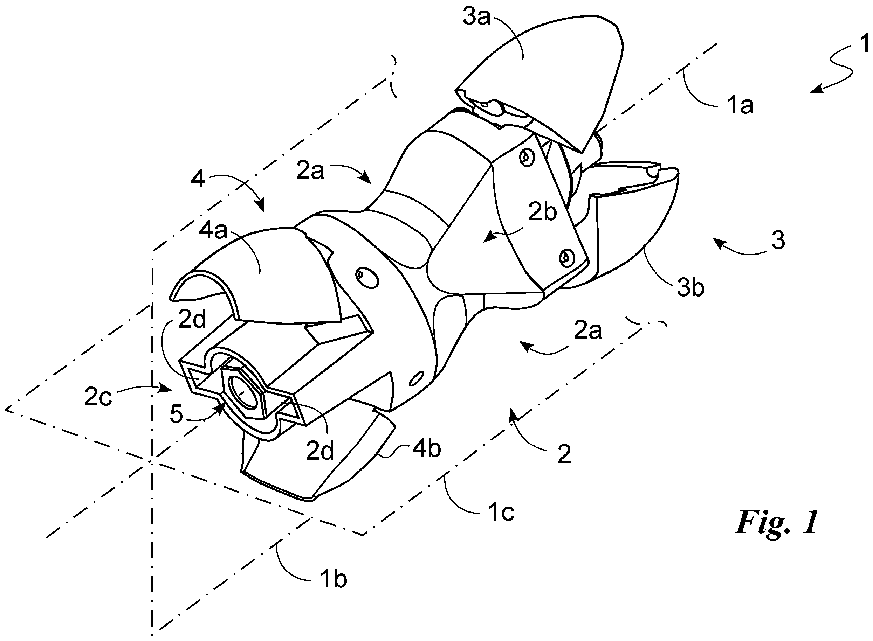

It defines a sagittal plane **1b** that is, suitably, barycentric; and/or a frontal plane 1c that is, suitably, barycentric (Fig. 1).

The sagittal plane 1b is perpendicular to the frontal plane 1c.

The intersection between the sagittal plane 1b and the frontal plane 1c defines the axis 1a.

The distractor 1 comprises a base body **2** that is designed to be placed between two spinous processes by spacing them apart.

The base body 2 defines, in relation to the longitudinal axis 1a, a first end and a second end.

The base body 2 has a sagittal section (i.e. defined by the intersection of the sagittal plane 1b with the base body 2) with at least one convexity defining a depression **2a** housing a spinous process.

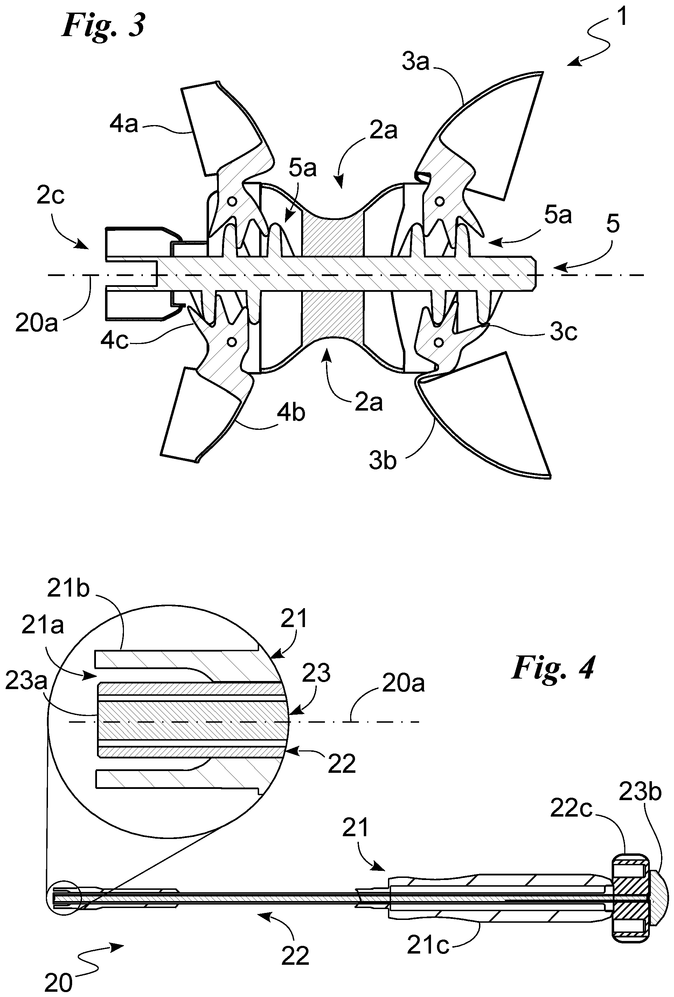

In detail, and as illustrated in Fig. 3, said sagittal section has two convexities and, therefore, two depressions 2a placed opposite the frontal plane 1c and each one is designed to accommodate a spinous process. The depressions 2a preferably is specular to the frontal plane 1c and, therefore, the sagittal section is symmetrical in relation to the frontal plane 1c.

The base body 2 has the minimum sagittal height at the bottom of the at least one depression 2a and, preferably, the maximum sagittal height at the apex of the at least one depression 2a.

In this document, the expression "sagittal height" identifies a length calculated perpendicular to the frontal plane 1c.

The minimum sagittal height of the base body 2 preferably is basically less than 100%, in detail less than 85%, and in more detail less than 65% of the maximum sagittal height of the same base body 2.

The profile of the depression 2a preferably is elliptical with a suitable eccentricity of less than 1, in detail less than 0.95, and in more detail basically ranging between 0.95 and 0.75, and preferably between 0.9 and 0.8.

The base body 2 has a frontal section (i.e. defined by the intersection of the frontal plane 1c with the base body 2) tapered with a minimum section close to the first end. In detail, the frontal section comprises a first proximal sector at the first end of a minimum and suitably constant frontal height; a second proximal sector at the second end of a maximum and suitably constant frontal height; and a central sector joining the first and second sectors and, thus, defining at least one slope **2b** between the first and second sectors.

In this document, the expression "frontal height" identifies a length calculated perpendicular to the sagittal plane 1b.

The central sector preferably has an increasing monotonic frontal height.

The at least one slope 2b is formed at the depressions 2a.

The radial section of the base body 2 made at the bottom of the depressions 2a has a frontal height and sagittal height basically equal to each other. Said radial section can preferably be circle-shaped.

The start of the slope 2b, i.e. the central sector, is at a shorter axial distance from the first end than the bottom of the depressions 2a.

In this document, the terms "radial" and "axial" identify a direction/plane respectively perpendicular and parallel to the longitudinal axis 1a.

The maximum frontal height and maximum sagittal height preferably is almost equal. The minimum frontal height preferably is less than the minimum sagittal height.

The central sector has two slopes 2b on opposite sides to the sagittal plane 1b and suitably almost specular to each other in relation to the sagittal plane 1b. Consequently, the central sector and, thus, the base body 2 preferably is specular to the sagittal plane 1b.

The base body 2 preferably is hollow.

The distractor 1 comprises a unit 3 for locking the distractor 1 at the spinous processes and, to be precise, between the spinous processes.

The unit 3 (also called herein anchoring unit or locking unit) is attached to the base body 2 at the first end and can be moved in relation to the body 2 so as to expand radially (in particular perpendicularly to the frontal plane 1c) locking the distractor 1 between the spinous processes.

The unit 3 is designed to define at least one expanded position wherein it has a maximum radial expansion; and at least one contracted position wherein it has a minimum radial expansion.

The unit 3 defines the head of the distractor 1 entering between the spinous processes.

The unit 3 preferably comprises at least one wing hinged to the base body 2 at the first end defining the rotation axis, which is, suitably, almost parallel to the frontal plane 1c. In detail, it comprises a first wing **3a** hinged to the base body 2 and a second wing **3b** hinged to the base body 2 on the opposite side to the first wing 3a in relation to the frontal plane 1c.

The wings 3a and 3b preferably has rotation axes that are basically parallel to each other.

The wings 3a and 3b preferably rotates simultaneously and in the opposite direction during a change in the position of the unit 3.

They preferably have a tapered axial profile with a minimum section distal to the base body 2 so that, in the contracted position, the locking unit 3 is axially tapered and, in particular, ogive.

The wings 3a and 3b preferably has a U-shaped radial section with an opening facing the longitudinal axis 1a.

The distractor 1 preferably comprises an additional unit **4** for locking the distractor 1 at the spinous processes and, to be precise, between the spinous processes.

The additional unit 4 is attached to the base body 2 at the second end and can be moved in relation to the body 2 so as to expand radially (in particular perpendicularly to the frontal plane 1c) locking the distractor 1 between the spinous processes. The additional unit 4 is designed to define an additional expanded position wherein it has maximum radial expansion; and an additional contracted position wherein it has minimum radial expansion.

The additional unit 4 preferably comprises at least one additional wing hinged to the base body 2 at the second end with the rotation axis suitably almost parallel to the frontal plane 1c. In detail, it comprises a first additional wing **4a** hinged to the base body 2 and a second additional wing **4b** hinged to the base body 2 on the opposite side to the first additional wing 4a in relation to the frontal plane 1c.

The additional wings 4a and 4b have rotation axes basically parallel to each other and, in detail, to the wings 3a and 3b.

The additional wings 4a and 4b rotate simultaneously and in the opposite direction during a change in the position of the additional unit 4.

The distractor 1 comprises an actuator **5** designed to control the passage of the distractor 1 between an anchoring configuration and an un-anchoring configuration.

In the anchoring configuration, the unit 3 is in the expanded position locking the distractor 1 in the correct position; and, suitably, the additional unit 4 is in the additional expanded position locking the distractor 1 on the opposite side of the unit 3 in relation to the depression 2a.

In the un-anchoring configuration, the unit 3 is in the contracted position and not locking the distractor 1 in the correct position; and, suitably, the additional unit 4 is in the additional contracted position.

The actuator 5 is designed to rotate, specifically exclusively, about the longitudinal axis 1a in relation to the base body 2 by controlling a change in the configuration of the distractor 1.

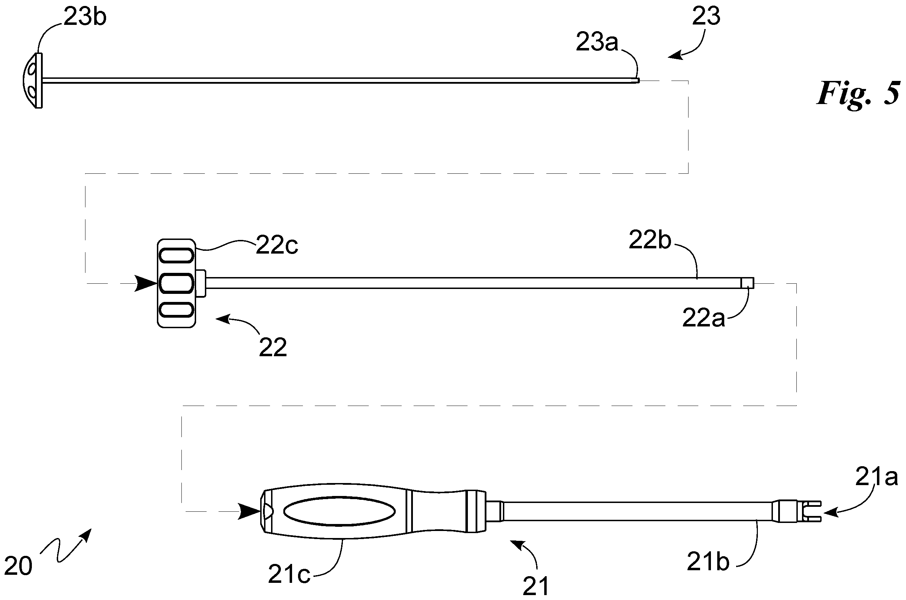

The actuator 5, shown in Fig. 5, preferably comprises a control thread **5a** of the unit 3 and, suitably, an additional control thread **5b** of the additional unit 4.

The additional thread 5b preferably is opposite the thread 5a.

To this end, the unit 3 preferably comprises, for each wing 3a and/or 3b, at least one thread 5a engagement tooth **3c** that, when pushed by the actuator 5, controls the rotation of the wings 3a and/or 3b.

The additional unit 4 preferably comprises, for each additional wing 5a and/or 5b, at least one additional thread 5b engagement tooth **4c** that, when pushed by the actuator 5, controls the rotation of the additional tooth 4c by controlling the rotation of the additional wings 4a and/or 4b.

The actuator 5 is externally controlled and the base body 2 comprises an opening 2c for accessing the actuator 5 at the second end.

The opening 2c is always in view, i.e. accessible from the outside, regardless of the configuration of the distractor 1. The additional unit 4 in the contracted position is, therefore, folded over the base body 2 leaving the opening 2c in view.

The actuator 5 is at least partially housed in the base body 2.

The distractor 1 can be part of an interspinous vertebral distraction device **10.**

The distraction device 10 comprises the distractor 1, previously described, and a control **20** for the distractor 1.

The control 20, as presented in Figs. 4 and 5, defines a main extension axis **20a** that is preferably barycentric.

It is designed to be engaged with the distractor 1 at the opening 2c suitably arranging the axes 1a and 20a basically parallel to each other and, more specifically, so that they basically coincide.

The control 20 comprises a first tool **21** for controlling the rotation of the distractor 1.

The first tool 21 is designed to define a first rotational constraint with, suitably exclusively, the base body 2 i.e. a constraint that prevents their mutual rotation about the axes 1a and/or 20a.

It comprises first means **21a** designed to be engaged with the body 2, suitably at the opening 2c, defining said first rotational constraint.

The first means 21a preferably comprises at least one tip and the base body 2 preferably comprises at least one seat **2d** for inserting said tip. In particular, they comprise two tips located on opposite sides of the axis 1a and the base body 2 comprises a seat 2d for each tip.

The first tool 21 comprises a hollow channel **21b** and, thus, defines a channel designed to be placed in communication with the opening 2c when the first tool 21 is attached to the base body 2.

The channel 21a is parallel to the main extension axis 20a.

The first means 21b protrude from the channel 21b along the main extension axis 20a.

The first tool 21 comprises a grip **21c** connected to the channel 21b on the opposite side to the means 21a.

The control 20 preferably comprises a second tool **22** for controlling the change in configuration of the distractor 1.

The second tool 22 is designed to define a second rotational constraint with, suitably exclusively, the actuator 5 i.e. a constraint that prevents their mutual rotation about the axes 1a and/or 20a.

It comprises second means **22a** designed to be engaged with the actuator 5, suitably at the opening 2c, defining said second rotational constraint

The second constraint is basically an interlocking mechanism. For example, the actuator 5 preferably comprises a polygonal (e.g. hexagonal) end that is in view of the opening 2c and the second means 22a preferably defines a counter-shaped engagement seat at said end.

The second tool 22 preferably rotates in relation to the base body 2 and the first tool 21 about the extension axis 20a.

It can be inserted into the channel 21a.

The second tool 22 preferably comprises an additional hollow channel **22b** and define an additional channel designed to be placed in communication with the opening 2c when the second tool 22 is attached to the actuator 5.

The additional channel 22a is parallel to the main extension axis 20a.

The second tool 22 preferably comprises a head **22c** for controlling the second tool 22.

The head 22c is designed to be placed outside the first tool 21 when the second tool 22 is in the channel 21b.

The control **20** preferably comprises a third tool **23** axially constraining at least one of the tools 21 and 22 to the distractor 1.

The third tool 23 is designed to be engaged with the actuator 5 by defining said axial constraint and, thus, preventing axial motion of the first tool 21 and/or of the second tool 22 in relation to the distractor 1.

It preferably comprises third means **23a** defining said axial constraint.

The third tool 23 preferably comprises a gripping element **23b** for controlling the third tool 23.

The gripping element 23b is designed to be placed outside the first tool 21 and the second tool 22 when the third tool 23 is in the additional channel 22b.

The third means 23a preferably defines a threaded coupling with the actuator 5. The third tool 23 can be at least partially arranged in the additional channel.

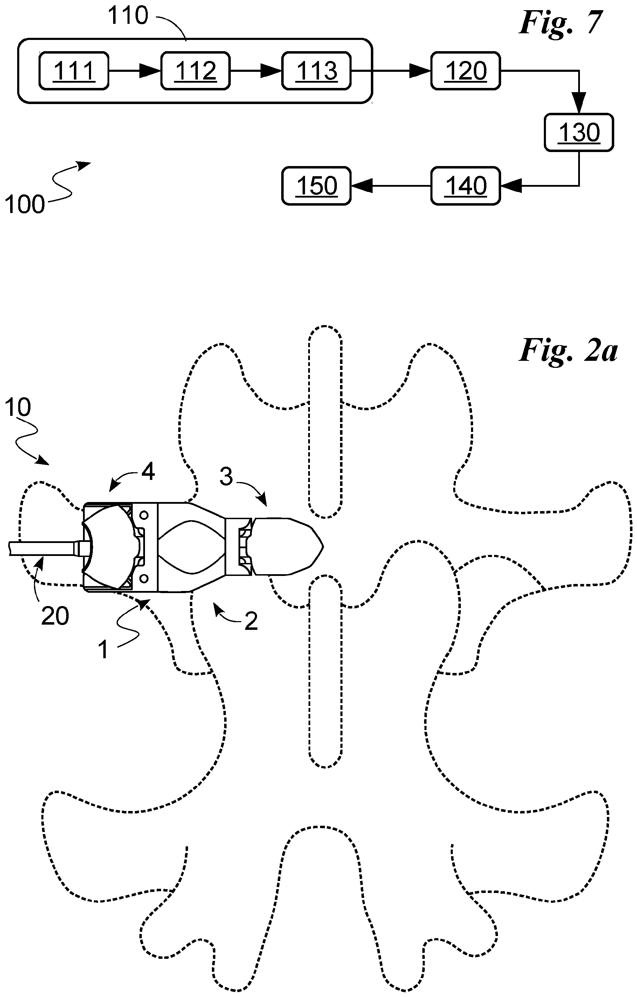

The operation of the distractor 1 and of the distraction device 10, described above in structural terms, define an innovative interspinous vertebral distraction procedure **100** that (not claimed) is schematised in Fig. 7.

The procedure 100 comprises the distractor 1 and, in particular, the distraction device 10.

The procedure 100 preferably comprises a preparation step **110** in which the control 20 is attached to the distractor 1.

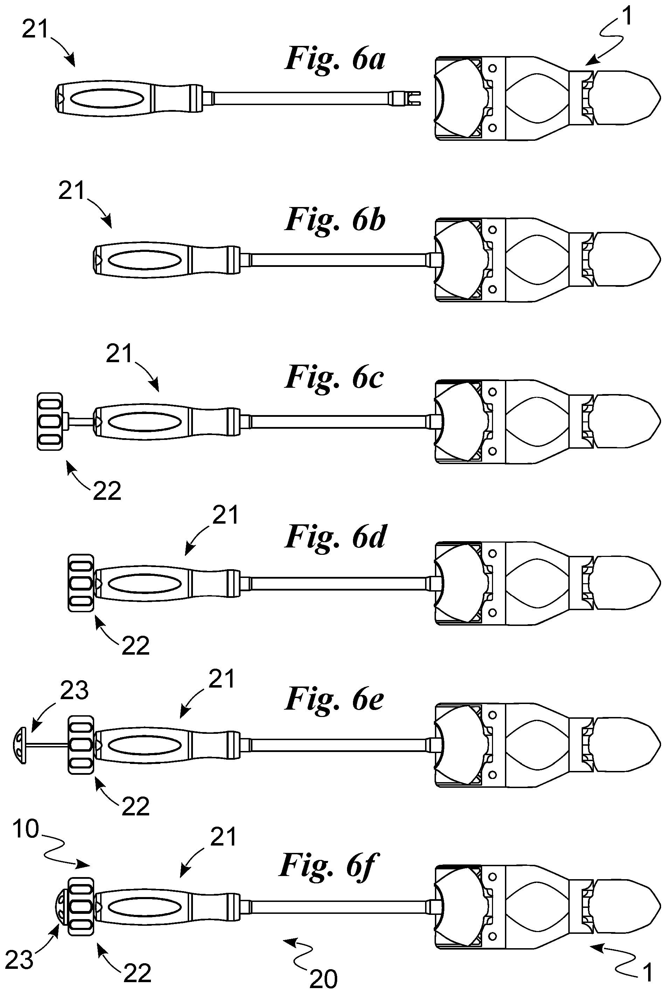

The preparation step 110 preferably comprises a first constraint sub-step **111** (Figs. 6a-6b) wherein the first tool 21 is engaged to the base body 2 defining said first rotational constraint. In detail, the first constraint is implemented thanks to the first means 21a by inserting the at least one tip in the at least one seat 2b.

The preparation step 110 preferably comprises a second constraint sub-step **112** (Figs. 6c-6d) wherein the second tool 22 is engaged with the actuator 5 defining said second rotational constraint. In detail, the first constraint is implemented by coupling the second means 22a to the actuator 5 by interference.

In the sub-step 112, the second tool 22 is inserted into the channel 21b.

The preparation step 110 preferably comprises a third constraint sub-step **113** (Figs. 6d-6e) wherein the third tool 23 is designed to be engaged to the actuator 5 defining said axial constraint.

At the end of the preparation step 110, the distractor 1 is in the un-anchoring configuration.

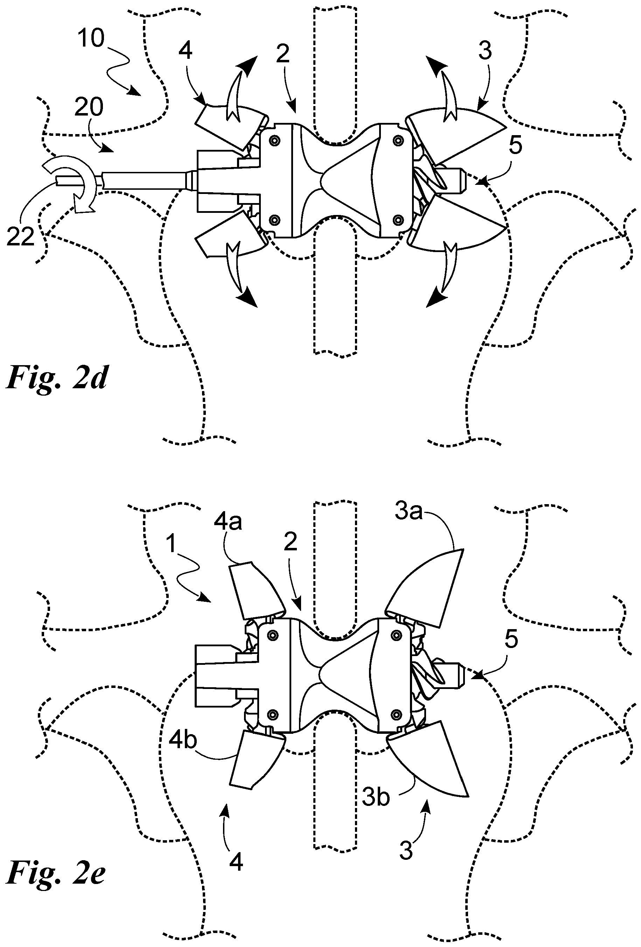

The procedure 100 preferably comprises a positioning step **120** (Figs. 2a-2b) wherein the distractor 1, suitably controlled by the control 20 and, in particular, by the first tool 21, is brought near the spinous processes.

In this step 120, the distractor 1 is placed with the frontal plane 1c basically perpendicular to the patient's frontal plane and then moved along the longitudinal axis 1a bringing the locking unit 3 basically to the two spinous processes. The distractor 1 then moves axially forward so that the unit 3, in the contracted position, penetrates between the spinous processes and, thanks to its tapered profile, is arranged between them.

The step 120 ends when the at least one slope 2b is proximal to the spinous processes.

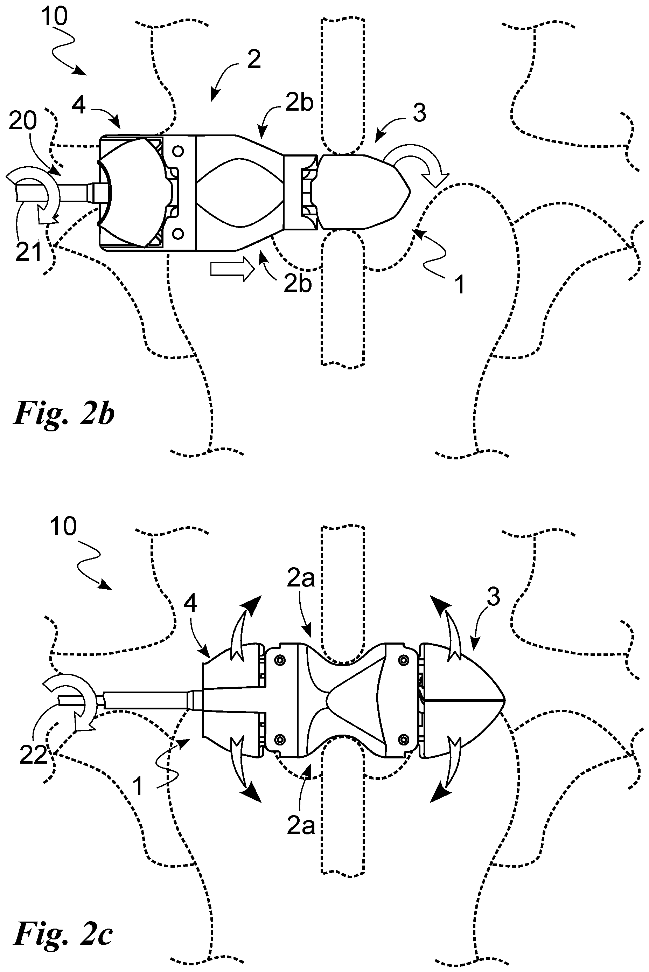

The procedure 100 preferably comprises an insertion step **130** (Figs. 2b-2c) wherein the base body 2 and, in detail, the entire distractor 1 rotate about the longitudinal axis 1a arranging the frontal plane 1c basically parallel to the patient's frontal plane and, thus, the two spinous processes in the depressions 2a.

In this step 130, the first tool 21 rotates the distractor 1 and, to be precise, axially moves the distractor 1 and, preferably simultaneously, rotates it about the longitudinal axis 1a.

The axial translation causes the spinous processes to slide along the slopes 2b so that they gradually adapt to the profile of the sagittal section and, thus, to the at least one depression 2a.

The rotation enables the depressions 2a to appear and, thus, to house the spinous processes inside, suitably after they have travelled at least part of the slopes 2b.

During the insertion step 130, the distractor 1 is rotated by an angle basically ranging between 60° and 120°, in detail between 75° and 105°, and preferably around 90°.

The procedure 100 preferably comprises an expansion step **140** (Figs. 2c-2d) wherein the distractor 1 switches from the anchoring configuration to the un-anchoring configuration.

In the expansion step 140, the actuator 5, controlled by the second tool 22, causes the unit 3 to pass into the expanded position so as to constrain the distractor 1 between the spinous processes.

At the same time, the actuator 5 suitably controls the passage of the additional unit 4 into the additional expanded position so as to constrain the distractor 1 between the spinous processes on the opposite side to the unit 3 in relation to the depressions 2a.

It should be noted that the variation in the configuration of the distractor 1 is controlled by rotating the second tool 22 in relation to the first tool 21 that, suitably, remains substantially still.

The procedure 100 preferably comprises a removal step **150** (Fig. 2e) for moving the control 20 away from the distractor 1.

In this step 150, the third tool 23 is disengaged from the actuator 5; then the second tool 23 and then the first tool 21 are moved away from the distractor 1.

The distractor 1, the vertebral distraction device 10 and, thus, the interspinous vertebral distraction procedure 100 (not claimed) according to the invention achieve important advantages.

In fact, thanks to the particular conformation of the distractor 1 and/or the practicality of the distractor control defined by control 20, they are particularly simple and quick to use and, in particular, enable a precise, firm, and rapid positioning of the distractor 1 between the spinous processes.

An important advantage is, therefore, the high stability of the distractor 1 once implanted. In fact, thanks to the depressions 2a, for example, the distractor 1 has a precise housing for each spinous process.

These advantages are enhanced by the presence of slopes 2b that, by gradually spacing the spinous processes apart, make it possible to carry out both a rapid and precise positioning of the distractor 1 and a less invasive procedure for the patient. Another important advantage is the particular control 20 that makes it possible to have a precise and practical control of the distractor 1 and, in particular, of the wings 3a and 3b and, suitably, of the additional wings 4a and 4b.

A significant advantage is that the distractor 1 (and, thus, the vertebral distraction device 10 and procedure 100) have low costs.

## Claims

1. Interspinous distraction device (10) comprising:
- an interspinous vertebral distractor (1) defining a longitudinal axis (1a); and
- a control (20) for said distractor (1);
- said interspinous vertebral distractor (1) comprising
∘ a base body (2) designed to be placed between two spinous processes and defining, in relation to said longitudinal axis (1a), a first end and a second end;
∘ a locking unit (3) attached to said base body (2) at said first end and designed to expand constraining said distractor (1) at said two spinous processes;
∘ an actuator (5) for controlling the passage of said locking unit (3) between an expanded position wherein said locking unit (3) constrains said interspinous vertebral distractor (1) between said two spinous processes and a contracted position wherein said locking unit (3) does not constrain said interspinous vertebral distractor (1) between said two spinous processes;
- said base body (2) comprising an opening (2c) for access to said actuator (5) at said second end;
said control (20) comprising
- a first tool (21) comprising a hollow channel (21a) and designed to attach itself rotationally to said base body (2) at said second end by arranging said hollow channel (21a) in communication with said opening (2c) so as to control a rotation of said interspinous vertebral distractor (1) in relation to said spinous processes;
- a second tool (22) designed to control, through said actuator (5), a change in said locking unit (3);
- said second tool (22) is being designed to be inserted in said hollow channel (21a) and rotated in relation to said first tool (21) controlling said locking unit (3);
**characterised in that**
- said interspinous vertebral distractor (1) defines a sagittal plane (1b) and a frontal plane (1c); wherein a frontal section of said base body (2) comprises a first sector, a second sector, and a central sector; wherein said first sector is at said first end and has a minimum height, wherein said second sector is at said second end and has a maximum height; wherein said central sector defines at least one slope (2b) joining said first sector and said second sector; and wherein said base body (2) has a sagittal section comprising at least one convexity defining at least one depression (2a) formed at said at least one slope (2b).

2. The interspinous distraction device (10) according to the previous claim, wherein said first tool (21) is designed to rotationally attach itself exclusively to said base body (2); and wherein said second tool (22) is designed to rotationally attach itself exclusively to said actuator (5).

3. The interspinous distraction device (10) according to at least one of claims 1-2, wherein said control (20) comprises a third tool (23) designed to axially attach said tools (21, 22) to said interspinous vertebral distractor (1); wherein said second tool (22) comprises an additional hollow channel (22a) designed to accommodate said third tool (23); wherein said third tool (23) is designed to be attached to said actuator (5).

4. The interspinous distraction device (10) according to at least one of claims 1-3, wherein said first tool (21) comprises at least one tip; and wherein said base body (2) comprises at least one seat (2d) for inserting of said tip.

5. The interspinous distraction device (10) according to at least one of claims 1-4, wherein said second tool (22) comprises a control head (22c) of said second tool (22) designed to be placed outside said first tool (21) when said second tool (22) is in said hollow channel (21a).

## Patentansprüche

1. Interspinöses Distraktionsgerät (10), umfassend:
- einen interspinösen Wirbeldistraktor (1), der eine Längsachse (1a) definiert; und
- eine Steuerung (20) für den Distraktor (1);
- der interspinöse Distraktor (1) umfassend:
∘ einen Basiskörper (2), der ausgelegt ist, zwischen zwei Dornfortsätzen positioniert zu werden, und der in Bezug auf die Längsachse (1a) ein erstes Ende und ein zweites Ende definiert;
∘ eine Verriegelungseinheit (3), die an dem Basiskörper (2) an dem ersten Ende angebracht wird und die ausgelegt ist, sich zu verbreiten, indem sie den Distraktor (1) an den beiden Dornfortsätzen befestigt;
∘ einen Aktuator (5) zum Steuern des Übergangs der Verriegelungseinheit (3) zwischen einer verbreiteten Position, in der die Verriegelungseinheit (3) den interspinösen Distraktor (1) zwischen den beiden Dornfortsätzen befestigt, und einer kontrahierten Position, in der die Verriegelungseinheit (3) den interspinösen Distraktor (1) nicht zwischen den beiden Dornfortsätzen befestigt;
- wobei der Basiskörper (2) eine Öffnung (2c) zum Zugang zu dem Aktuator (5) an dem zweiten Ende umfasst;
Die Steuerung (20) umfassend:
- ein erstes Werkzeug (21) umfassend einen Hohlkanal (21a) und das ausgelegt ist, sich drehbar an dem Basiskörper (2) an dem zweiten Ende anzubringen, indem der Hohlkanal (21a) in Verbindung mit der Öffnung (2c) gesetzt wird, um eine Drehung des interspinösen Distraktors (1) in Bezug auf die Dornfortsätze zu steuern;
- ein zweites Werkzeug (22), das ausgelegt ist, durch den Aktuator (5) eine Änderung der Verriegelungseinheit (3) zu steuern;
- wobei das zweite Werkzeug (22) ausgelegt ist, in den Hohlkanal (21a) eingeführt zu werden und in Bezug auf das erste Werkzeug (21) gedreht zu werden, sodass die Verriegelungseinheit (3) gesteuert wird;
**dadurch gekennzeichnet, dass**
- der interspinöse Wirbeldistraktor (1) eine Sagittalebene (1b) und eine Frontbene (1c) definiert; wobei ein Frontabschnitt des Basiskörpers (2) einen ersten Abschnitt, einen zweiten Abschnitt und einen zentralen Abschnitt umfasst; wobei der erste Abschnitt an dem ersten Ende liegt und eine Minimalhöhe aufweist; wobei der zweite Abschnitt an dem zweiten Ende liegt und eine Maximalhöhe aufweist; wobei der zentrale Abschnitt mindestens eine Neigung (2b) definiert, die den ersten Abschnitt und den zweiten Abschnitt verbindet; und wobei der Basiskörper (2) einen Sagittalabschnitt aufweist, der mindestens eine Konvexität umfasst, die mindestens eine Vertiefung (2a) definiert, die an der mindestens einen Neigung (2b) ausgebildet ist.

2. Interspinöses Distraktionsgerät (10) nach dem vorhergehenden Anspruch, wobei das erste Werkzeug (21) ausgelegt ist, sich drehbar ausschließlich an dem Basiskörper (2) anzubringen; und wobei das zweite Werkzeug (22) ausgelegt ist, sich drehbar ausschließlich an dem Aktuator (5) anzubringen.

3. Interspinöses Distraktionsgerät (10) nach mindestens einem der Ansprüche 1-2, wobei die Steuerung (20) ein drittes Werkzeug (23) umfasst, das ausgelegt ist, die Werkzeuge (21, 22) axial an dem interspinösen Distraktor (1) anzubringen; wobei das zweite Werkzeug (22) einen zusätzlichen Hohlkanal (22a) umfasst, der zur Aufnahme des dritten Werkzeugs (23) ausgelegt ist; wobei das dritte Werkzeug (23) ausgelegt ist, an dem Aktuator (5) angebracht zu werden.

4. Interspinöses Distraktionsgerät (10) nach mindestens einem der Ansprüche 1-3, wobei das erste Werkzeug (21) mindestens eine Spitze umfasst; und wobei der Basiskörper (2) mindestens einen Sitz (2d) zum Einführen der Spitze umfasst.

5. Interspinöses Distraktionsgerät (10) nach mindestens einem der Ansprüche 1-4, wobei das zweite Werkzeug (22) einen Steuerkopf (22c) des zweiten Werkzeugs (22) umfasst, der ausgelegt ist, außerhalb des ersten Werkzeugs (21) platziert zu werden, wenn sich das zweite Werkzeug (22) in dem Hohlkanal (21a) befindet.

## Revendications

1. Dispositif de distraction interépineux (10) comprenant:
- un distracteur interépineux (1) définissant un axe longitudinal (1a); et
- un contrôle (20) pour ledit distracteur (1);
- ledit distracteur interépineux (1) comprenant:
∘ un corps de base (2) conçu pour être placé entre deux processus épineux et définissant, par rapport audit axe longitudinal (1a), une première extrémité et une seconde extrémité;
∘ une unité de verrouillage (3) fixée audit corps de base (2) au niveau de ladite première extrémité et conçue pour se dilater, contraignant ainsi ledit distracteur (1 4-a) contre lesdits deux processus épineux;
∘ un actionneur (5) pour contrôler le passage de ladite unité de verrouillage (3) entre une position déployée, dans laquelle ladite unité de verrouillage (3) contraint ledit distracteur interépineux (1) entre lesdits deux processus épineux, et une position contractée, dans laquelle ladite unité de verrouillage (3) ne contraint pas ledit distracteur interépineux (1) entre lesdits deux processus épineux;
- ledit corps de base (2) comprenant une ouverture (2c) pour accéder audit actionneur (5) au niveau de ladite seconde extrémité;
Ledit contrôle (20) comprenant:
- un premier outil (21) comprenant un canal creux (21 a) et conçu pour se fixer de manière rotative audit corps de base (2) au niveau de ladite seconde extrémité en disposant ledit canal creux (21 a) en communication avec ladite ouverture (2c), de manière à contrôler la rotation dudit distracteur interépineux (1) par rapport auxdits processus épineux;
- un second outil (22) conçu pour contrôler, par l'intermédiaire dudit actionneur (5), une modification de ladite unité de verrouillage (3);
- ledit second outil (22) étant conçu pour être inséré dans ledit canal creux (21a) et être mis en rotation par rapport audit premier outil (21), contrôlant ainsi ladite unité de verrouillage (3);
**Caractérisé en ce que**
- ledit distracteur interépineux (1) définit un plan sagittal (1 b) et un plan frontal (1 c); où une section frontale dudit corps de base (2) comprend un premier secteur, un second secteur et un secteur central; où ledit premier secteur est situé au niveau de ladite première extrémité et présente une hauteur minimale; où ledit second secteur est situé au niveau de ladite seconde extrémité et présente une hauteur maximale; où ledit secteur central définit au moins une pente (2b) reliant ledit premier secteur et ledit second secteur; et **en ce que** ledit corps de base (2) présente une section sagittale comprenant au moins une convexité définissant au moins une dépression (2a) formée au niveau de ladite au moins une pente (2b).

2. Dispositif de distraction interépineux (10) selon la revendication précédente, dans lequel ledit premier outil (21) est conçu pour se fixer de manière rotative exclusivement audit corps de base (2); et dans lequel ledit second outil (22) est conçu pour se fixer de manière rotative exclusivement audit actionneur (5).

3. Dispositif de distraction interépineux (10) selon au moins l'une des revendications 1-2, dans lequel ledit contrôle (20) comprend un troisième outil (23) conçu pour fixer axialement lesdits outils (21, 22) audit distracteur interépineux (1); dans lequel ledit second outil (22) comprend un canal creux supplémentaire (22a) conçu pour loger ledit troisième outil (23); dans lequel ledit troisième outil (23) est conçu pour être fixé audit actionneur (5).

4. Dispositif de distraction interépineux (10) selon au moins l'une des revendications 1-3, dans lequel ledit premier outil (21) comprend au moins une pointe; et dans lequel ledit corps de base (2) comprend au moins un logement (2d) pour l'insertion de ladite pointe.

5. Dispositif de distraction interépineux (10) selon au moins l'une des revendications 1-4, dans lequel ledit second outil (22) comprend une tête de contrôle (22c) dudit second outil (22) conçue pour être placée à l'extérieur dudit premier outil (21) lorsque ledit second outil (22) est dans ledit canal creux (21a).
